(19) Europäisches Patentamt
European Patent Office
Office européen des brevets

(11) **EP 3 785 660 B1**

(12) **EUROPEAN PATENT SPECIFICATION**

(45) Date of publication and mention
of the grant of the patent:
**07.06.2023   Bulletin 2023/23**

(21) Application number: **19791730.5**

(22) Date of filing: **29.04.2019**

(51) International Patent Classification (IPC):
*A61B 34/32* *(2016.01)*     *A61B 34/37* *(2016.01)*
*A61B 17/02* *(2006.01)*

(52) Cooperative Patent Classification (CPC):
**A61B 34/37; A61B 34/32;** A61B 17/02;
A61B 90/361

(86) International application number:
**PCT/CN2019/084889**

(87) International publication number:
**WO 2019/206340 (31.10.2019 Gazette 2019/44)**

(54) **SURGICAL ROBOT SYSTEM**

CHIRURGISCHES ROBOTERSYSTEM

SYSTÈME DE ROBOT CHIRURGICAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **27.04.2018   CN 201810395743**

(43) Date of publication of application:
**03.03.2021   Bulletin 2021/09**

(73) Proprietor: **Shanghai Microport Medbot (Group)
Co., Ltd.
Shanghai 201203 (CN)**

(72) Inventors:
• **WANG, Jiayin**
  **Shanghai 201203 (CN)**
• **HE, Chao**
  **Shanghai 201203 (CN)**
• **SHI, Yunlei**
  **Shanghai 201203 (CN)**
• **YUAN, Shuai**
  **Shanghai 201203 (CN)**
• **ZHU, Xiang**
  **Shanghai 201203 (CN)**

(74) Representative: **Patentship
Patentanwaltsgesellschaft mbH
Elsenheimerstraße 65
80687 München (DE)**

(56) References cited:
**EP-A1- 3 260 051          CN-A- 102 905 641
CN-A- 105 877 891        CN-A- 108 420 538
CN-U- 206 825 435        KR-B1- 101 742 534
US-A1- 2012 182 134      US-A1- 2017 265 865
US-B1- 9 833 254          US-B2- 9 668 821**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**TECHNICAL FIELD**

The present invention relates to the technical field of medical devices and, more specifically, to a surgical robot system.

**BACKGROUND**

Minimally invasive surgery using a surgical robot system has the advantages of less trauma, rapid recovery, less wound infection for patient, and less difficulty in surgical operation and less fatigue for surgeon. The surgical robot system ensures that the surgeon has a strong sense of immersion at the same time.

When a surgeon performs a surgical operation, it is first required to accurately cut and suture the diseased tissue; secondly, a tool is required to pull the tissue which will be removed or turned over, so that the tissue after cutting is smooth with less bleeding and no obvious burn, facilitating the postoperative recovery of the patient; finally, when the tissue needs to be removed or turned over, the tool for pulling the tissue is required to be adjusted to cause a desired traction force according to the progress of the operation.

Patent publication CN106028994A provides a surgical robot system with four arms for surgical operations, and the fourth arm of the system can only perform the pulling operation in the fixed position of the tissue. Therefore, the surgical robot cannot follow the treatment of the diseased tissue during a surgical operation, so that the cutting surface of the tissue is rough with more bleeding and obvious burn marks during the cutting process, which prolongs the process of wound healing, and increases the patient's recovery time and surgical cost.

Patent publication CN107427327A relates to a configurable robotic surgical system with a virtual trajectory and a flexible endoscope. When the user moves a single robot arm among the robot arms, the other robot arms will automatically move to maintain the virtual trajectory alignment. It can achieve a convenient control of the robot arms, which however is not helpful for smoothing the cut of the tissue, less bleeding, or no obvious burn marks in surgical operations.

Patent publication CN108472087A relates to a system and method for a variable-velocity surgical instrument in a computer-assisted medical device. The computer-assisted medical device is configured to set a velocity set point of the actuator to an initial velocity and monitor force or torque applied by the actuator. When the applied force or torque is above a force or torque limit, it is determined whether a continue condition for the operation is satisfied. When the continue condition is satisfied, the operation is paused and when the continue condition is not satisfied, it is determined whether forced firing of the actuator should take place. The system and method are not helpful in smoothing the cut of the tissue, less bleeding, and no obvious burn marks during surgical operations.

Furthermore, EP 3 260 051 A1 discloses a support arm apparatus which is provided with a base unit, an arm unit and a control device, wherein a retractor is provided on the arm unit and an actuator is provided to drive the arm unit and the retractor to move, wherein the driving of the actuator is controlled by the control device and the retractor is used to hold an organ of a patient during surgery. US 2012/182134 A1 discloses a positioning system for holding an instrument in surgical procedures and providing multiple degrees of freedom of movement to the instrument. US 9 833 254 B1 discloses a robotic surgical system that includes a surgical instrument configured to cut tissues and an imaging system configured to image the tissues. KR 101 742 534 B1 discloses a surgical instrument, in particular a multi-directional retractor for pulling tissues of a patent.

**SUMMARY OF THE INVENTION**

The present application is set out in the appended set of claims.

This application provides a surgical robot system, comprising an execution terminal, wherein the execution terminal includes a first tool arm provided with a first surgical instrument, a second tool arm provided with a second surgical instrument, a driving device configured to drive the first tool arm, the first surgical instrument, the second tool arm and the second surgical instrument to move, and a control unit communicatively connected with the driving device, wherein

the first tool arm and the first surgical instrument are configured to pull tissues and organs;
the second tool arm and the second surgical instrument are configured to perform a surgical operation on tissues and organs;
the control unit is configured to obtain a magnitude of a Cartesian force exerted by the tissues and organs on the first surgical instrument, and compare the magnitude of the Cartesian force exerted by the tissues and organs on the first surgical instrument with a preset force value; if the magnitude of the Cartesian force exerted by the tissues and organs on the first surgical instrument is less than the preset force value, the control unit controls the driving device to drive the first tool arm and the first surgical instrument according to the preset force value, to increase a traction force exerted by the first surgical instrument on the tissues and organs to the preset force value.

Optionally, in the surgical robot system, if the magnitude of the Cartesian force exerted by the tissues and organs on the first surgical instrument is less than the preset force value, the control unit takes a direction from a preset point of action to a point of action of the Cartesian force exerted by the tissues and organs on the first surgical instrument as a direction of a preset force, and controls the driving device to drive the first tool arm and the first surgical instrument according to the preset force, to increase the traction force exerted by the first surgical instrument on the tissues and organs to the preset force value.

Optionally, in the surgical robot system, the preset point of action is at a position where the second surgical instrument initially acts on the tissues and organs, or

the preset point of action is at a position where the second surgical instrument acts on the tissues and organs when the control unit obtains the preset point of action.

Optionally, in the surgical robot system, the execution terminal further comprises a third tool arm provided with a third surgical instrument; the third tool arm and the third surgical instrument are configured to clamp tissues and organs, and the preset point of action is at a position where the third surgical instrument acts on the tissues and organs.

Optionally, in the surgical robot system, the execution terminal further comprises a force sensor communicatively connected with the control unit, and the force sensor is configured to sense the Cartesian force exerted by the tissues and organs on the first surgical instrument.

Optionally, in the surgical robot system, when the second surgical instrument starts to perform a surgical operation on the tissues and organs, the control unit obtains the magnitude of the Cartesian force exerted by the tissues and organs on the first surgical instrument, and compares the preset force value with the magnitude of the Cartesian force exerted by the tissues and organs on the first surgical instrument, or

the control unit obtains the magnitude of the Cartesian force exerted by the tissues and organs on the first surgical instrument always, when the second surgical instrument starts to perform a surgical operation on the tissues and organs, the control unit compares the preset force value with the magnitude of the Cartesian force exerted by the tissues and organs on the first surgical instrument.

Optionally, in the surgical robot system,

the control unit is also configured to obtain a magnitude of a Cartesian force received by the second surgical instrument, and compare the magnitude of the Cartesian force received by the second surgical instrument with a force threshold; if the magnitude of the Cartesian force received by the second surgical instrument is greater than the force threshold, the control unit determines that the second surgical instrument starts to perform a surgical operation on the tissues and organs; or

the control unit is also configured to obtain a magnitude of a Cartesian force received by the second surgical instrument, and compare a difference between two obtained Cartesian forces received by the second surgical instrument with a force threshold; if the difference between the two obtained Cartesian forces received by the second surgical instrument is greater than the force threshold, the control unit determines that the second surgical instrument starts to perform a surgical operation on the tissues and organs.

Optionally, in the surgical robot system,

the execution terminal further comprises a first position sensor communicatively connected with the control unit, and the first position sensor is configured to sense a position of the first tool arm and/or a position of the first surgical instrument,

the control unit is configured to obtain a position of the point of action of the Cartesian force exerted by the tissues and organs on the first surgical instrument according to the position of the first tool arm and/or the position of the first surgical instrument.

Optionally, in the surgical robot system,

the execution terminal further comprises a first position sensor communicatively connected with the control unit, and the first position sensor is configured to sense a position of the second tool arm and/or a position of the second surgical instrument,

the control unit is configured to obtain a position where the second surgical instrument acts on the tissues and organs according to the position of the second tool arm and/or the position of the second surgical instrument.

Optionally, in the surgical robot system,

the execution terminal further comprises a third tool arm provided with a third surgical instrument; the third surgical instrument and the third tool arm are configured to pull tissues and organs,
the preset point of action includes a first preset point of action and a second preset point of action;
the control unit is configured to compare the magnitude of the Cartesian force exerted by the tissues and organs on the first surgical instrument with the preset force value; if the magnitude of the Cartesian force exerted by the tissues and organs on the first surgical instrument is less than the preset force value, the control unit takes a direction from the first preset point of action to a point of action of the Cartesian force exerted by the tissues and organs on the first surgical instrument as a direction of a preset force, and controls the driving device to drive the first tool arm and the first surgical instrument according to the preset force, to increase the traction force exerted by the first surgical instrument on the tissues and organs to the preset force value;
the control unit is configured to compare a magnitude of the Cartesian force exerted by the tissues and organs on the third surgical instrument with the preset force value; if the magnitude of the Cartesian force exerted by the tissues and organs on the third surgical instrument is less than the preset force value, the control unit takes a direction from the second preset point of action to a point of action of the Cartesian force exerted by the tissues and organs on the third surgical instrument as a direction of a preset force, and controls the driving device to drive the third tool arm and the third surgical instrument according to the preset force, to increase a traction force exerted by the third surgical instrument on the tissues and organs to the preset force value;
the first preset point of action is at a position where the third surgical instrument acts on the tissues and organs, and the second preset point of action is at a position where the first surgical instrument acts on the tissues and organs.

Optionally, in the surgical robot system,

the execution terminal further comprises a torque sensor communicatively connected with the control unit; the first tool arm comprises a plurality of first tool arm joints, and the first surgical instrument comprises a plurality of first surgical instrument joints; the torque sensor is arranged on each of the first tool arm joints and the first surgical instrument joints to sense a moment of force received by each of the first tool arm joints and the first surgical instrument joints resulting from a deformation in a static state;
the control unit is further configured to obtain from the torque sensor an initial moment of force received by each of the first tool arm joints and the first surgical instrument joints when the preset force is determined;
the control unit is further configured to obtain from the torque sensor a current moment of force received by each of the first tool arm joints and the first surgical instrument joints, then obtain an increment of the moment of force received by each of the first tool arm joints and the first surgical instrument joints according to the initial moment of force and the obtained current moment of force to further obtain a command moment of force on each of the first tool arm joints and the first surgical instrument joints, and control the driving device to drive the first tool arm joint and the first surgical instrument joint according to the command moment of force, to increase the traction force exerted by the third surgical instrument on the tissues and organs to the preset force value.

Optionally, in the surgical robot system,

the execution terminal further comprises a second position sensor communicatively connected with the control unit, and the second position sensor is configured to sense a position of each of the first tool arm joints and the first surgical instrument joints;
the control unit is configured to obtain an increment of the position of each of the first tool arm joints and the first surgical instrument joints according to the increment of the moment of force received by each of the first tool arm joints and the first surgical instrument joints and a control stiffness of each of the first tool arm joints and the first surgical instrument joints, and to further obtain a command position of each of the first tool arm joints and the first surgical instrument joints according to a current position and the obtained increment of the position of each of the first tool arm joints and the first surgical instrument joints, to obtain a Jacobian matrix in the command position;
the control unit is configured to obtain a traction force exerted by the first surgical instrument on the tissues and organs in the command position according to the Jacobian matrix and the command moment of force on each of the first tool arm joints and the first surgical instrument joints;
the control unit is configured to compare a magnitude of the traction force exerted by the first surgical instrument on the tissues and organs in the command position with the preset force value; if a difference between the magnitude of the traction force exerted by the first surgical instrument on the tissues and organs in the command position and the preset force value is greater than a tolerance value, the control unit adjusts the increment of the moment of force received by the first tool arm joint and the first surgical instrument joint, so that the difference between the magnitude of the traction force exerted by the first surgical instrument on the tissues and organs in the command position and the preset force value is less than the tolerance value.

Optionally, in the surgical robot system,
the execution terminal further includes an imaging arm provided with an endoscope, and the preset force value is determined by using the endoscope to observe a state of pulled tissues and organs.

Optionally, in the surgical robot system, the surgical robot system further comprises a control terminal, wherein the control terminal comprises a master manipulator configured to control a movement of the first tool arm and the first surgical instrument, and receive the Cartesian force exerted by the tissues and organs on the first surgical instrument; and the preset force value is determined based on the Cartesian force exerted by the tissues and organs on the first surgical instrument when the tissues and organs are tensed.

In the surgical robot system proposed in the present application, the first tool arm and the first surgical instrument are configured to pull tissues and organs, the second tool arm and the second surgical instrument are configured to perform a surgical operation on tissues and organs, the control unit is configured to obtain a magnitude of a Cartesian force exerted by the tissues and organs on the first surgical instrument, and compare the magnitude of the Cartesian force exerted by the tissues and organs on the first surgical instrument with a preset force value; if the Cartesian force exerted by the tissues and organs on the first surgical instrument is less than the preset force value, the control unit controls the driving device to drive the first tool arm and the first surgical instrument according to the preset force value, to increase a traction force exerted by the first surgical instrument on the tissues and organs to the preset force value. As a result, a function of autonomous pulling is achieved.

## BRIEF DESCRIPTION OF THE DRAWINGS

Fig. 1 is a schematic diagram showing a structure of a surgical robot system according to an embodiment of the present invention;

Fig. 2 is a schematic diagram showing the force generated in a pulling operation performed by one tool arm and a surgical instrument thereof according to an embodiment of the present application;

Fig. 3 is a schematic diagram showing the force generated in a pulling operation performed by one tool arm and a surgical instrument thereof according to another embodiment of the present application;

Fig. 4 is a schematic diagram showing the forces generated in a pulling operation performed by two tool arms and surgical instruments thereof according to an embodiment of the present application.

In the drawings,
10-vision cart; 11-bedside cart; 12-surgical cart; 13-tool cart; 20-surgeon console; 21-display device; 30-surgeon; 40-human tissues and organs; 110-imaging arm; 111-endoscope; 112-tool arms; 112a-first tool arm; 112b-second tool arm; 112c-third tool arm; 113-surgical instruments; 113a-first surgical instrument; 113b-second surgical instrument; 113c -third surgical instrument.

## DETAILED DESCRIPTION

The surgical robot system proposed in the present invention will be described in greater detail with reference to specific embodiments and the accompanying drawings. Features and advantages of the invention will be more apparent from the following detailed description, and from the appended claims. Note that the figures are much simplified and may not be drawn to scale, and their sole purpose is to facilitate easy and clear explanation of some embodiments of the invention. In addition, the structure shown in the drawings is in most cases a part of the actual structure. In particular, the focus of each drawing is different, and different scales are sometimes used.

This application provides a surgical robot system. The surgical robot system includes an execution terminal including a first tool arm mounted with a first surgical instrument, a second tool arm mounted with a second surgical instrument, a driving device for driving the first tool arm, the first surgical instrument, the second tool arm and the second surgical instrument to move, and a control unit communicatively connected with the driving device. The first tool arm and the first surgical instrument are configured for pulling human tissues and organs. The second tool arm and the second surgical instrument are configured to perform surgical operations on the human tissues and organs. The control unit is configured to obtain the magnitude of the Cartesian force exerted by the human tissues and organs on the first surgical instrument, and compare the magnitude of the Cartesian force exerted by the human tissues and organs on the first surgical instrument and a preset force value. If the magnitude of the Cartesian force exerted by the human tissues and organs on the first surgical instrument is less than the preset force value, the control unit controls the driving device to drive the first tool arm and the first surgical instrument according to the preset force value, so that the magnitude of the traction force exerted by the first surgical instrument on the human tissues and organs reaches the preset force value.

EMBODIMENT 1

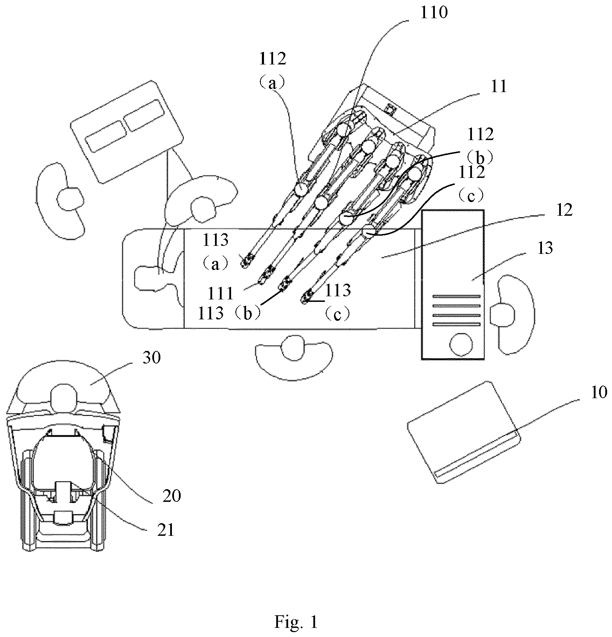

Fig. 1 is a schematic diagram showing a structure of a surgical robot system according to an embodiment. In the embodiment shown in Fig. 1, the surgical robot system includes an execution terminal and a control terminal. The execution terminal may include a vision cart 10, a bedside cart 11, a surgical cart 12 and a tool cart 13. The control terminal includes a surgeon console 20. The surgical robot system is mainly used to perform minimally invasive surgical treatment on patients on the surgical cart 12.

In this embodiment, the bedside cart 11 includes at least one imaging arm 110 mounted with an endoscope 111, such as a 3D laparoscope. The endoscope 111 is configured to obtain information about human tissues and organs, surgical instruments, and surgical environment.

In this embodiment, the bedside cart 11 further includes three tool arms 112 which are a first tool arm 112a, a second tool arm 112b, and a third tool arm 112c. Each tool arm 112 is respectively mounted with a surgical instrument 113. In other words, the first tool arm 112a is mounted with a first surgical instrument 113a, the second tool arm 112b is mounted with a second surgical instrument 113b, and the third tool arm 112c is mounted with a third surgical instrument 113c. The endoscope 111 and the surgical instrument 113 enter the body of the patient and arrive at the position of lesion through the wound on the patient's body.

Please continue to refer to Fig. 1, in the embodiment of this application, the surgeon console 20 includes a master manipulator (not shown in the figure). A master-slave control relationship is formed between the master manipulator and the tool arm 112 and the surgical instrument 113 in the execution terminal. Specifically, the tool arm 112 and the surgical instrument 113 move according to the movement of the main manipulator during the operation, that is, move according to the operation by the surgeon's hand. Further, the main manipulator also receives the information about the force exerted by the human tissues and organs on the surgical instrument and feeds it back to the surgeon's hand, so that the surgeon can more intuitively feel the surgical operation. The surgeon console 20 also includes a display device 21. The surgeon 30 can observe the internal environment of the patient and the movement of the end of the surgical instrument 113 through the display device 21 (which may be a two-dimensional display or a three-dimensional display) in the surgeon console 20.

In this embodiment, the first tool arm 112a and the first surgical instrument 113a are configured to pull human tissues and organs; the second tool arm 112b and the second surgical instrument 113b are configured to perform surgical operations (including tissue cutting, suture, peeling, etc.); and the third tool arm 112c and the third surgical instrument 113c are configured to clamp the human tissues and organs. Those skilled in the art should understand that the first tool arm 112a and the first surgical instrument 113a, the third tool arm 112c and the third surgical instrument 113c can also be configured to perform surgical operations (including tissue cutting, suture, peeling, etc.); the second tool arm 112b and the second surgical instrument 113b can also be configured to pull or clamp human tissues and organs; and the third tool arm 112c and the third surgical instrument 113c can also be configured to pull human tissues and organs. In other embodiments of the present application, the bedside cart 11 may also include only two tool arms 112 or more tool arms 112, for example, four tool arms 112 or five tool arms 112.

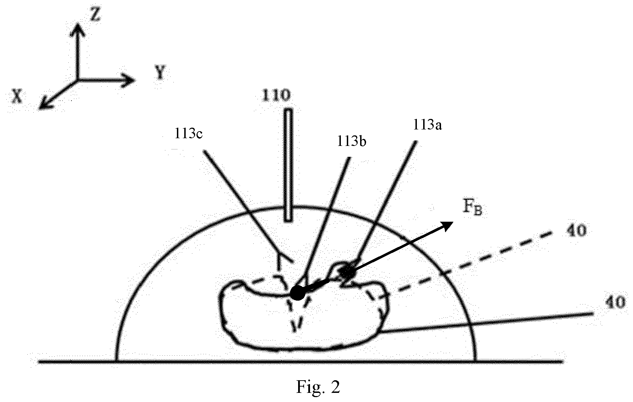

In this embodiment, the second tool arm 112b and the second surgical instrument 113b can perform cutting operations on human tissues and organs 40, and the first tool arm 112a and the first surgical instrument 113a are configured for pulling human tissues and organs. Due to the existence of the first tool arm 112a and the first surgical instrument 113a, it is easy to generate surface tension and internal tension in the human tissues and organs 40, as shown by the dotted line in Fig. 2. During the surgical operation, the surface tension ensures the "outward tension state" of the tissues at the point of action performed by the second surgical instrument 113b, the internal tension ensures the "continuous tension state" of the tissues during tissue cutting. However, as the cutting process goes on, the point of action performed on the human tissues and organs 40 by the second surgical instrument 113b will be changed in its position accordingly, and the Cartesian force exerted by the human tissues and organs 40 on the first surgical instrument 113a will also change. If the first tool arm 112a and the first surgical instrument 113a are not adjusted, the magnitude of the traction force exerted by the first surgical instrument 113a on the human tissues and organs 40 will be less than the preset force value, leading to a result that the human tissues and organs 40 is not tensed.

In the embodiment of the present application, the control unit is configured to receive the information about the Cartesian force received by the first surgical instrument 113a, and determines whether the first tool arm 112a and the first surgical instrument 113a need to be adjusted according to the preset force value, and control the traction force exerted by the first surgical instrument 113a on the human tissues and organs 40 to reach the preset force value. As a result, the human tissues and organs 40 are always tensed, and an autonomous pulling function of the surgical robot system is achieved.

Specifically, in the embodiment of the present application, the execution terminal further includes a force sensor (not shown in the figures) communicatively connected with the control unit, and the force sensor is configured to detect the Cartesian force exerted by the human tissues and organs 40 on the first surgical instrument 113a. The present embodiment does not particularly limit the location of the force sensor. For example, the force sensor is provided at the end of the first surgical instrument 113a.

The control unit is configured to compare the magnitude of the Cartesian force obtained from the force sensor with the preset force value, and if the preset force value is greater than the Cartesian force, it indicates that the human tissues and organs 40 are not tensed enough, the control unit can use a direction from a preset point of action to a point of action of the Cartesian force exerted by the human tissues and organs 40 on the first surgical instrument 113a as a direction of the preset force, and control the driving device according the preset force value and the preset direction to drive the first tool arm 112a and the first surgical instrument 113a so that the traction force exerted by the first surgical instrument 113a on the human tissues and organs 40 reaches the preset force value. In other words, the human tissues and organs 40 are tensed again from a state in which the human tissues and organs 40 are not tensed enough, thereby achieving the autonomous pulling function.

In this embodiment, no specific limitation is made to the ways of achieving an increase of the traction force exerted by the first surgical instrument 113a on the human tissues and organs 40 to reach the preset force, which is caused by the first tool arm 112a and the first surgical instrument 113a both driven by the driving device under the control of the control unit. For example, the first tool arm 112a includes n first tool arm joints (not shown in the figures), and the driving device acts on each of the $n$ first tool arm joints. The first surgical instrument 113a includes $m$ first surgical instrument joints (not shown in the figures), and the driving device also acts on each of the $m$ first surgical instrument joints. According to the following formula, the control unit calculates the moment of force received by each of the first tool arm joints and first surgical instrument joints (i.e., the required moment of force exerted by the driving device for driving each of the first tool arm joints and first surgical instrument joints) according to the expected Cartesian force $F_B$ (whose value equals to the value of the preset force, but direction opposite to the direction of the preset force) exerted by the human tissues and organs 40 on the first surgical instrument 113a:

$$\begin{bmatrix} \tau_1 \\ \bullet \\ \bullet \\ \tau_i \\ \bullet \\ \bullet \\ \tau_n \\ \tau'_1 \\ \bullet \\ \bullet \\ \tau'_j \\ \bullet \\ \bullet \\ \tau'_m \end{bmatrix} = J^T \times F_B \quad i = 1, 2, ..., n \quad j = 1, 2, ..., m$$

where, $F_B$ represents the expected Cartesian force exerted by the human tissues and organs 40 on the first surgical instrument 113a, n represents the number of the first tool arm joints of the first tool arm 112a, $\tau_i$ represents the moment of force received by the i-th first tool arm joint of the first tool arm 112a, $m$ represents the number of the first surgical instrument joints of the first surgical instrument 113a, $\tau'_j$ represents the moment of force received by the j-th first surgical instrument joint of the first surgical instrument 113a, J represents the kinematic Jacobian matrix of the current positions of the first tool arm 112a and the first surgical instrument 113a, that is, the partial derivative matrix of the position in Cartesian space with respect to the angle in joint of the surgical instrument end effector.

In other embodiments of the present application, the ends of the second surgical instrument 113b and the third surgical instrument 113c may also be provided with the force sensors to sense the Cartesian force exerted by the human tissues and organs 40 on the corresponding surgical instruments when the second surgical instrument 113b and the third surgical instrument 113c are configured to pull the human tissues and organs 40.

Further, when the second surgical instrument 113b starts to perform a surgical operation on the human tissues and organs 40, the control unit receives the information about the Cartesian force received by the first surgical instrument

113a and compares the Cartesian force received by the first surgical instrument 113a with the preset force value. In another case, the control unit receives the information about the Cartesian force received by the first surgical instrument 113a always. When the second tool arm 112b starts to perform a surgical operation on the human tissues and organs 40, the control unit compares the Cartesian force received by the first surgical instrument 113a with the preset force value. The embodiments of the present application do not limit the ways of determining whether or not the second surgical instrument 113b starts to perform a surgical operation on the human tissues and organs 40. For example, the end of the second surgical instrument 113b is provided with a force sensor communicatively connected with the control unit, and the control unit is configured to obtain the information about the Cartesian force received by the second surgical instrument 113b according to the sensing results of the force sensor. If the Cartesian force received by the second surgical instrument 113b exceeds a preset force threshold, it is determined that the second surgical instrument 113b starts to perform a surgical operation on the human tissues and organs 40. In another example, the control unit obtains twice the information about the Cartesian force received by the second surgical instrument 113b and compares the difference between the Cartesian forces received by the second surgical instrument 113b with the force threshold. If the difference between the Cartesian forces received by the second surgical instrument 113b is greater than the force threshold, it is determined that the second surgical instruments 113b starts to perform a surgical operation on the human tissues and organs.

Referring to Figs. 1 and 2, in the embodiment of the present application, the preset point of action is a point of action initially performed by the second surgical instrument 113b on the human tissues and organs 40. The present application does not limit the ways of obtaining the position of the point of action initially performed by the second surgical instrument 113b on the human tissues and organs 40 as well as the position of the point of action of the Cartesian force exerted by the human tissues and organs 40 on the first surgical instrument 113a. For example, the execution terminal further includes a first position sensor communicatively connected with the control unit, and the first position sensor is configured to sense the position of the first tool arm 112a and/or the position of the first surgical instrument 113a. Preferably, the first position sensor includes an optical target lens which is placed on the tool arm, the position and Euler angle of the optical target lens are measured by an optical tracker, and a position and posture of the optical target lens in the base coordinate system is obtained. According to the position and posture of the surgical instrument in the optical target lens coordinate system, the position and posture of the surgical instrument in the base coordinate system is obtained. For details, please refer to patent publication CN 105232155A.

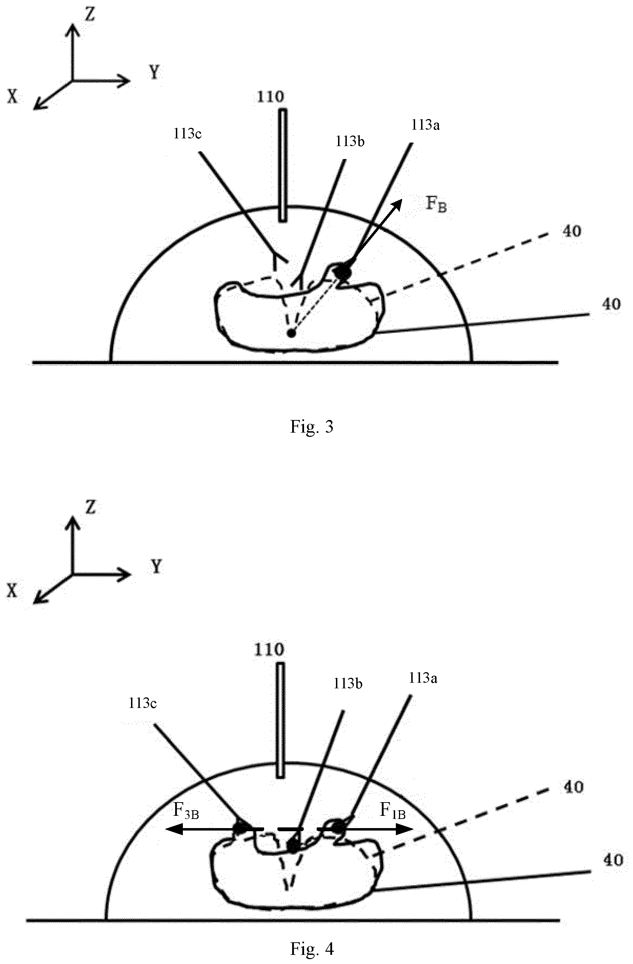

In another example, the first position sensor includes an angle sensor, and the angle sensor is configured to sense the positions of joints of the first tool arm 112a, the second tool arm 112b, the first surgical instrument 113a, and the second surgical instrument 113b. The position of the points of action (performed by the ends of the first surgical instruments and the second surgical instruments) in the Cartesian space (i.e., base coordinate system) is calculated from the positive kinematics (e.g., DH method). The dotted lines in Fig. 2 show the current state of the human tissues and organs 40, and the solid lines show the initial state of the human tissues and organs 40. As shown in Fig. 3, the position of the preset point of action can also be determined based on the current position of the point of action performed by the second surgical instrument 113b on the human tissues and organs 40 (i.e., the current position of the end of the second surgical instrument 113b). The "current" here refers to a time instant when the control unit obtains the preset point of action. At this time instant, the direction of the preset force is defined from the current position where the second surgical instrument 113b performs a surgical operation on the human tissues and organs 40 to the position where the first surgical instrument 113a pulls the human tissues and organs 40. If the execution terminal also includes a third tool arm 112c and a third surgical instrument 113c which are configured for clamping human tissues and organs, the preset point of action may also be the point of action performed by the third surgical instrument 113c on the human tissues and organs.

In this embodiment, the preset force value is determined by using by the endoscope 111 to observe the state of the pulled human tissues and organs. Before or during a surgical operation, in the case that the autonomous pulling function of the surgical robot system is not activated, the surgeon pulls the human tissues and organs 40 through the tool arms and surgical instruments, and determines the preset force value by observing the tension state of the pulled human tissues and organs. In another case, the preset force value may also be an empirical value obtained by a surgeon comprehensively according to multiple surgical factors such as the type of operation, the type of tissues and organs, and the gender and age of the patient.

Further, the surgeon console 20 also includes a lock switch for autonomous pulling, so that the control unit can be notified to turn on and off the autonomous pulling function of the robot system. Before the preset force value is determined, the surgeon console 20 can notify the control unit to turn off the autonomous pulling function of the robot system through the lock switch, and therefore the control unit does not obtain the information about the Cartesian force received by the first surgical instrument 113a, or the control unit obtains the information about the Cartesian force received by the first surgical instrument 113a but does not compare the Cartesian force received by the first surgical instrument 113a with the preset force, or the control unit compares the Cartesian force received by the first surgical instrument 113a with the preset force but does not control the drive device to drive. After the preset force value is determined, the surgeon console 20 can, through the lock switch, notify the control unit to activate the autonomous pulling function of the robot system, so that the magnitude of the traction force exerted by the first surgical instrument 113a on the human tissues and organs reaches the preset force value.

Therefore, in this embodiment, the control unit obtains the information about the Cartesian force received by the first surgical instrument 113a, and the human tissues and organs are ensured to have a reasonable and constant tension, and the sustainability of the tension of the lesion tissue is ensured. The surgical operation performed by the second surgical instrument 113b can be completed with a small operational motion, and the incision therefore is smooth with less bleeding. The surgical operation is easy to perform, especially, on the tissues and organs with great thickness, such as the kidney.

EMBODIMENT 2

Please continue to refer to Figs. 1 and 4, different from the above embodiment, the third tool arm 112c and the third surgical instrument 113c in this embodiment are also configured to pull the human tissues and organs 40, and the control unit is configured to compare the magnitude of the Cartesian force exerted by the human tissues and organs 40 on the third surgical instrument 113c with the preset force value. If the preset force value is greater than the magnitude of the Cartesian force exerted by the human tissues and organs 40 on the third surgical instrument 113c, the control unit uses a direction from a preset point of action to a point of action of the Cartesian force exerted by the human tissues and organs 40 on the third surgical instrument 113c as a preset direction of force, and controls the driving device to drive the joints of the third tool arm 112c and third surgical instrument 113c according to the preset force value and the preset direction of force, so that the magnitude of the traction force exerted by the third surgical instrument 113c on the human tissues and organs 40 reaches the preset force value.

In the case that two tool arms and two surgical instruments are configured to pull the human tissues and organs 40, the first surgical instrument 113a and the third surgical instrument 113c can be provided with a force sensor respectively on their ends to sense the Cartesian forces exerted by the human tissues and organs 40 on the first surgical instrument 113a and the third surgical instrument 113c respectively. As a result, the traction forces exerted respectively by the first surgical instrument 113a and the third surgical instrument 113c on the human tissues and organs 40 can be obtained.

The control unit then compares the Cartesian forces exerted by the human tissues and organs 40 on the first surgical instrument 113a and the third surgical instrument 113c with the preset force value respectively. If the preset force value is greater, the control unit controls the driving device to drive the first tool arm 112a, the first surgical instrument 113a, the third tool arm 112c and the third surgical instrument 113c, so that the magnitude of the traction force exerted by the first surgical instrument 113a on the human tissues and organs 40 and the magnitude of the traction force exerted by the third surgical instrument 113c on the human tissues and organs 40 reach the preset force value.

In this embodiment, the preset force value may be determined by the feeling of the surgeon about the force applied to the surgeon by the master manipulator. In other words, before or during the surgical operation, the surgical system is in a pulling state, and the surgeon uses the surgical instruments to pull the human tissues and organs 40 so that the pulled human tissues and organs 40 are tensed. At this time, the master manipulator receives the Cartesian forces exerted by the human tissues and organs 40 on the surgical instruments, and feedbacks it to the surgeon. The surgeon determines the preset force value according to the feedback force.

If the Cartesian force exerted by the human tissues or organs on the surgical instruments are less than the preset force value, it means that the human tissues and organs 40 are not tensed, and the control unit needs to drive the first tool arm 112a, the first surgical instrument 113a, the third tool arm 112c and the third surgical instrument 113c to adjust the force exerted by the first surgical instrument 113a and the third surgical instrument 113c on the human tissues and organs 40. As shown in Fig. 4, in this embodiment, for the first tool arm 112a and the first surgical instrument 113a, the position of the preset point of action is determined according to the position of the end of the third surgical instrument 113c mounted on the third tool arm 112c, that is, the direction of the preset force $F_{1B}$ is defined from the point of action where the third surgical instrument 113c pulls the human tissues and organs 40 to the point of action where the first surgical instrument 113a pulls the human tissues and organs 40. For the third tool arm 112c and the third surgical instrument 113c, the position of the preset point of action is determined according to the end of the first surgical instrument 113a mounted on the first tool arm 112a, that is, the direction of the preset force $F_{3B}$ is defined from the point of action where the first surgical instrument 113a pulls the human tissues and organs 40 to the point of action where the third surgical instrument 113c pulls the human tissues and organs 40. In this case, the preset point of action includes a first preset point of action and a second preset point of action. The first preset point of action is at a point where the third surgical instrument 113c acts on the human tissues and organs, and the second preset point of action is at a point where the first surgical instrument 113a acts on the human tissues and organs. The dotted lines in Fig. 4 show the current state of the human tissues and organs 40, and the solid lines show the initial state of the human tissues and organs 40. Similar to the above embodiment, no limitation is made to the ways of obtaining the position of the point of action where the third surgical instrument 113c pulls the human tissues and organs 40, and the position of the point of action where the first surgical instrument 113a pulls the human tissues and organs 40. The position of the points of action (performed by the ends of the first surgical instruments and the third surgical instruments) in the Cartesian space (i.e., base coordinate system) can be calculated from the positive kinematics (e.g., DH method) based on the positions, sensed by the angle sensor, of the joints of the first tool arm 112a, the first surgical instrument 113a, the third tool arm 112c and the third surgical instrument 113c.

After the directions of the preset forces for the first surgical instrument 113a and the third surgical instrument 113c are obtained, the control unit obtains the moment of force required by the driving device for driving each of the joints according to the preset force values, the directions of the preset forces and the Jacobian matrix mentioned above, and controls the driving device to drive the first tool arm joint, the first surgical instrument, the third tool arm joint and the third surgical instrument, so that the magnitudes of the traction forces respectively exerted by the first surgical instrument 113a and the third surgical instrument 113c on the human tissues and organs 40 reach the preset force values. In other words, the human tissues and organs 40 are tensed again from an untensioned state, so that the autonomous pulling function is achieved.

In this embodiment, the first tool arm 112a and the first surgical instrument 113a are configured to pull the human tissues and organs, and the second tool arm 112b and the second surgical instrument 113b are configured to perform surgical operations (including tissue cutting, stitching, peeling, etc.), the third tool arm 112c and the third surgical instrument 113c are also configured to pull the human tissues and organs 40. Those skilled in the art should understand that the first tool arm 112a and the first surgical instrument 113a can also be configured to perform surgical operations (including tissue cutting, suture, peeling, etc.), while the other two tool arms and two surgical instruments are configured to pull the human tissues and organs. Alternatively, the third tool arm 112c and the third surgical instrument 113c can also be configured to perform surgical operations (including tissue cutting, suture, peeling, etc.), while the other two tool arms and two surgical instruments are configured to pull the human tissues and organs. The bedside cart 11 may also include more tool arms 112 and surgical instruments 113 mounted thereon, for example, four tool arms 112 or five tool arms 112.

EMBODIMENT 3

Different from the first embodiment, the execution terminal in this embodiment includes a plurality of torque sensors and a second position sensor that are communicatively connected with the control unit. The first tool arm 112a includes n first tool arm joints (not shown in the figures). The first surgical instrument includes *m* first surgical instrument joints (not shown in the figures). The torque sensor is configured to sense the moment of force received by each of the first surgical instrument joints and first tool arm joints of the first tool arm 112a which is generated due to deformation in a static state. The second position sensor is configured to sense the position of each first tool arm joint and the position of each first surgical instrument joint.

When the surgeon determines that the tissues are tensed properly, the preset force $F_{ini}$ is determined accordingly, and each of the joints (the first tool arm joint and the first surgical instrument joint are simply written as joints hereinafter for the purpose of a simple description since the information about the moment of force and the position of both the first tool arm joint and the first surgical instrument joint are required) is locked while the first tool arm 112a and the first surgical instrument 113a remain stationary. At this time, the torque sensor mounted at each joint senses the moment of force caused by the deformation of the joint resulting from the Cartesian force. This moment is the initial moment. The torque sensor of the i-th joint may have corresponding readings $\tau_{i\_ini}$. The control unit obtains the initial amount of each joint from the torque sensor of each joint.

As the second surgical instrument 113b performs a cutting operation on the tissue, the tissue is bound to be in a relaxed state (untensioned state). At this time, the moment of force sensed by the torque sensor of the i-th joint is $\tau_i$. The control unit obtains the current moment of force on each joint from the torque sensor of each joint, and then obtains the increment of the moment of force on the i-th joint $\Delta\tau_i$, where $\Delta\tau_i = k_i{}^*(\tau_{i\_ini} - \tau_i)$, where $0 < k_i \leq 1$.

Further, based on the above increment $\Delta\tau_i$, the control unit can obtain the command moment of force on the i-th joint $\tau_{i\_cmd}$, where $\tau_{i\_cmd} = -(\Delta\tau_i + \tau_i)$, where the negative sign represents the opposite direction. Further, based on the increment $\Delta\tau_i$, the control unit obtains the position increment of the i-th joint $\Delta\theta_i$, where $\Delta\theta_i = \Delta\tau_i / k_{pi}$, where $k_{pi}$ represents the control stiffness of each joint. The control unit obtains the command position of the i-th joint $\theta_{i\_cmd}$, where $\theta_{i\_cmd} = \theta_i + \Delta\theta_i$, where the current position $\theta_i$ of the i-th joint is sensed by the second position sensor. According to the command position of each joint $\theta_{i\_cmd}$, the control unit obtains the kinematic Jacobian matrix $J_{cmd}{}^T$ of the first tool arm 112a and the first surgical instrument 113a at the command position through kinematic calculation, Further, the traction force $F_{cmd}$ exerted by the first surgical instrument 113a on the human tissues and organs at the command position is obtained according to:

$$F_{cmd} = \left(J_{\_cmd}^{T}\right)^{-1} \times \begin{bmatrix} \tau_{1\_cmd} \\ \bullet \\ \bullet \\ \tau_{i\_cmd} \\ \bullet \\ \bullet \\ \tau_{m+n\_cmd} \end{bmatrix} \qquad i = 1, 2, ..., m+n$$

Further, the control unit compares the modulus of the traction force $F_{cmd}$ exerted by the first surgical instrument 113a on the human tissues and organs with the modulus of the preset force $F_{ini}$. If the absolute value of the difference between the above two is less than a certain tolerance value, the control unit notifies the driving device to adjust each joint according to the command moment of force $\tau_{i\_cmd}$ on each joint; if it is greater than the tolerance value, the control unit readjusts the coefficient $k_i^{'}$, where $k_i^{'} = k_i + j*(1-k_i)/step$, where $j = 1,...,step$, step represents the step size, regains the increment $\Delta\tau_i$, where $\Delta\tau_i = k_i^{'}*(\tau_{i\_ini} - \tau_i)$, and then obtains a new traction force until the difference between the modulus of the traction force $F_{cmd}$ exerted by the first surgical instrument on the human tissues and organs and the modulus of the preset force $F_{ini}$ is less than the tolerance value.

In summary, in the surgical robot system proposed in the embodiments of the present application, the first tool arm and the first surgical instrument are configured to pull human tissues and organs, the second tool arm and the second surgical instrument are configured for performing surgical operations on human tissues and organs. The control unit includes a preset force value, and the control unit compares the magnitude of the Cartesian force exerted by the human tissues and organs on the first surgical instrument with the preset force value. If the preset force value is greater than the magnitude of the Cartesian force exerted by the human tissues and organs on the first surgical instrument, the control unit controls the driving device to drive the first tool arm and the first surgical instrument according to the preset force value, so that the magnitude of the traction force exerted by the first surgical instrument on the human tissues and organs reaches the preset force value. Thus, the control unit adjusts the magnitude of the traction force exerted by the first surgical instrument on the human tissues and organs according to the preset force value, so that the magnitude of the traction force exerted by the first surgical instrument on the human tissues and organs reaches the preset force value. As a result, the autonomous pulling function of the surgical robot system is achieved.

The description presented above merely describes some preferred embodiments of the present invention and does not limit the scope thereof in any sense. The invention is defined solely by the appended claims.

**Claims**

1. A surgical robot system, comprising an execution terminal, wherein the execution terminal includes a first tool arm (112a) provided with a first surgical instrument (113a), a second tool arm (112b) provided with a second surgical instrument (113b), a driving device configured to drive the first tool arm (112a), the first surgical instrument (113a), the second tool arm (112b) and the second surgical instrument (113b) to move, and a control unit communicatively connected with the driving device, wherein

   the first tool arm (112a) and the first surgical instrument (113a) are configured to pull tissues and organs;
   the second tool arm (112b) and the second surgical instrument (113b) are configured to perform a surgical operation on tissues and organs;
   the control unit is configured to obtain a magnitude of a Cartesian force exerted by the tissues and organs on the first surgical instrument (113a), and compare the magnitude of the Cartesian force exerted by the tissues and organs on the first surgical instrument (113a) with a preset force value;

   **characterised in that** if the magnitude of the Cartesian force exerted by the tissues and organs on the first surgical instrument (113a) is less than the preset force value, the control unit controls the driving device to drive the first tool arm (112a) and the first surgical instrument (113a) according to the preset force value, to increase a traction force exerted by the first surgical instrument (113a) on the tissues and organs to the preset force value and achieve autonomous pulling.

2. The surgical robot system according to claim 1, wherein if the magnitude of the Cartesian force exerted by the tissues and organs on the first surgical instrument (113a) is less than the preset force value, the control unit takes a direction from a preset point of action to a point of action of the Cartesian force exerted by the tissues and organs on the first surgical instrument (113a) as a direction of a preset force, and controls the driving device to drive the first tool arm (112a) and the first surgical instrument (113a) according to the preset force, to increase the traction force exerted by the first surgical instrument (113a) on the tissues and organs to the preset force value.

3. The surgical robot system according to claim 2, wherein the preset point of action is at a position where the second surgical instrument (113b) initially acts on the tissues and organs, or
the preset point of action is at a position where the second surgical instrument (113b) acts on the tissues and organs when the control unit obtains the preset point of action.

4. The surgical robot system according to claim 2, wherein the execution terminal further comprises a third tool arm (112c) provided with a third surgical instrument (113c); the third tool arm (112c) and the third surgical instrument (113c) are configured to clamp tissues and organs, and the preset point of action is at a position where the third surgical instrument (113c) acts on the tissues and organs.

5. The surgical robot system according to claim 1 or 2, wherein the execution terminal further comprises a force sensor communicatively connected with the control unit, and the force sensor is configured to sense the Cartesian force exerted by the tissues and organs on the first surgical instrument (113a).

6. The surgical robot system according to claim 1, wherein when the second surgical instrument (113b) starts to perform a surgical operation on the tissues and organs, the control unit obtains the magnitude of the Cartesian force exerted by the tissues and organs on the first surgical instrument (113a), and compares the preset force value with the magnitude of the Cartesian force exerted by the tissues and organs on the first surgical instrument (113a), or
the control unit obtains the magnitude of the Cartesian force exerted by the tissues and organs on the first surgical instrument (113a) always, when the second surgical instrument (113b) starts to perform a surgical operation on the tissues and organs, the control unit compares the preset force value with the magnitude of the Cartesian force exerted by the tissues and organs on the first surgical instrument (113a).

7. The surgical robot system according to claim 6, wherein

the control unit is also configured to obtain a magnitude of a Cartesian force received by the second surgical instrument (113b), and compare the magnitude of the Cartesian force received by the second surgical instrument (113b) with a force threshold; if the magnitude of the Cartesian force received by the second surgical instrument (113b) is greater than the force threshold, the control unit determines that the second surgical instrument (113b) starts to perform a surgical operation on the tissues and organs; or
the control unit is also configured to obtain a magnitude of a Cartesian force received by the second surgical instrument (113b), and compare a difference between two obtained Cartesian forces received by the second surgical instrument (113b) with a force threshold; if the difference between the two obtained Cartesian forces received by the second surgical instrument (113b) is greater than the force threshold, the control unit determines that the second surgical instrument (113b) starts to perform a surgical operation on the tissues and organs.

8. The surgical robot system according to claim 2, wherein

the execution terminal further comprises a first position sensor communicatively connected with the control unit, and the first position sensor is configured to sense a position of the first tool arm (112a) and/or a position of the first surgical instrument (113a),
the control unit is configured to obtain a position of the point of action of the Cartesian force exerted by the tissues and organs on the first surgical instrument (113a) according to the position of the first tool arm (112a) and/or the position of the first surgical instrument (113a).

9. The surgical robot system according to claim 3, wherein

the execution terminal further comprises a first position sensor communicatively connected with the control unit, and the first position sensor is configured to sense a position of the second tool arm (112b) and/or a position of the second surgical instrument (113b),

the control unit is configured to obtain a position where the second surgical instrument (113b) acts on the tissues and organs according to the position of the second tool arm (112b) and/or the position of the second surgical instrument (113b).

10. The surgical robot system according to claim 2, wherein

the execution terminal further comprises a third tool arm (112c) provided with a third surgical instrument (113c); the third surgical instrument (113c) and the third tool arm (112c) are configured to pull tissues and organs, the preset point of action includes a first preset point of action and a second preset point of action; wherein, if the magnitude of the Cartesian force exerted by the tissues and organs on the first surgical instrument (113a) is less than the preset force value,

the control unit takes a direction from the first preset point of action to a point of action of the Cartesian force exerted by the tissues and organs on the first surgical instrument (113a) as a direction of a preset force, and then continues to control the driving device to drive the first tool arm (112a) and the first surgical instrument (113a) according to the preset force, to increase the traction force exerted by the first surgical instrument (113a) on the tissues and organs to the preset force value;

the control unit is further configured to compare a magnitude of the Cartesian force exerted by the tissues and organs on the third surgical instrument (113c) with the preset force value; if the magnitude of the Cartesian force exerted by the tissues and organs on the third surgical instrument (113c) is less than the preset force value, the control unit takes a direction from the second preset point of action to a point of action of the Cartesian force exerted by the tissues and organs on the third surgical instrument (113c) as a direction of a preset force, and controls the driving device to drive the third tool arm (112c) and the third surgical instrument (113c) according to the preset force, to increase a traction force exerted by the third surgical instrument (113c) on the tissues and organs to the preset force value;

the first preset point of action is at a position where the third surgical instrument (113c) acts on the tissues and organs, and the second preset point of action is at a position where the first surgical instrument (113a) acts on the tissues and organs.

11. The surgical robot system according to claim 1, further comprising a control terminal, wherein the control terminal comprises a master manipulator configured to control a movement of the first tool arm (112a) and the first surgical instrument (113a), and receive the Cartesian force exerted by the tissues and organs on the first surgical instrument (113a); and the preset force value is determined based on the Cartesian force exerted by the tissues and organs on the first surgical instrument (113a) when the tissues and organs are tensed.

**Patentansprüche**

1. Chirurgisches Robotersystem, umfassend ein Ausführungsterminal, wobei das Ausführungsterminal einen ersten Werkzeugarm (112a), der mit einem ersten chirurgischen Instrument (113a) versehen ist, einen zweiten Werkzeugarm (112b), der mit einem zweiten chirurgischen Instrument (113b) versehen ist, eine Antriebsvorrichtung, die ausgebildet ist, den ersten Werkzeugarm (112a), das erste chirurgische Instrument (113a), den zweiten Werkzeugarm (112b) und das zweite chirurgische Instrument (113b) zum Bewegen anzutreiben, und eine Steuereinheit umfasst, die kommunikativ mit der Antriebsvorrichtung verbunden ist, wobei

der erste Werkzeugarm (112a) und das erste chirurgische Instrument (113a) ausgebildet sind, Gewebe und Organe zu ziehen; wobei der zweite Werkzeugarm (112b) und das zweite chirurgische Instrument (113b) ausgebildet sind, eine chirurgische Operation an Geweben und Organen durchzuführen; wobei die Steuereinheit ausgebildet ist, eine Größe einer kartesischen Kraft zu erhalten, die von den Geweben und Organen auf das erste chirurgische Instrument (113a) ausgeübt wird, und die Größe der kartesischen Kraft, die von den Geweben und Organen auf das erste chirurgische Instrument (113a) ausgeübt wird, mit einem voreingestellten Kraftwert zu vergleichen; **dadurch gekennzeichnet, dass** wenn die Größe der von den Geweben und Organen auf das erste chirurgische Instrument (113a) ausgeübte kartesische Kraft kleiner als der voreingestellte Kraftwert ist, die Steuereinheit die Antriebsvorrichtung ansteuert, um den ersten Werkzeugarm (112a) und das erste chirurgische Instrument (113a) entsprechend dem voreingestellten Kraftwert anzutreiben, um eine durch das erste chirurgische Instrument (113a) auf die Gewebe und Organe ausgeübte Zugkraft auf den voreingestellten Kraftwert zu erhöhen und ein autonomes Ziehen zu erreichen.

2. Chirurgisches Robotersystem nach Anspruch 1, wobei, wenn die Größe der kartesischen Kraft, die von den Geweben und Organen auf das erste chirurgische Instrument (113a) ausgeübt wird, kleiner als der voreingestellte Kraftwert ist, die Steuereinheit eine Richtung von einem voreingestellten Wirkpunkt zu einem Wirkpunkt der kartesischen Kraft, die von den Geweben und Organen auf das erste chirurgische Instrument (113a) ausgeübt wird, als eine Richtung einer voreingestellten Kraft zugrunde legt, und die Antriebsvorrichtung ansteuert, um den ersten Werkzeugarm (112a) und den das erste chirurgische Instrument (113a) gemäß der voreingestellten Kraft anzutreiben, um die durch das erste chirurgische Instrument (113a) auf die Gewebe und Organe ausgeübte Zugkraft auf den voreingestellten Kraftwert zu erhöhen.

3. Chirurgisches Robotersystem nach Anspruch 2, wobei der voreingestellte Wirkpunkt an einer Position ist, an der das zweite chirurgische Instrument (113b) initial auf die Gewebe und Organe einwirkt, oder wobei der voreingestellte Wirkpunkt an einer Position ist, an der das zweite chirurgische Instrument (113b) auf die Gewebe und Organe einwirkt, wenn die Steuereinheit den voreingestellten Wirkpunkt erhält.

4. Chirurgisches Robotersystem nach Anspruch 2, wobei das Ausführungsterminal ferner einen dritten Werkzeugarm (112c) umfasst, der mit einem dritten chirurgischen Instrument (113c) versehen ist; wobei der dritte Werkzeugarm (112c) und das dritte chirurgische Instrument (113c) ausgebildet sind, Gewebe und Organe zu klemmen, und wobei der voreingestellte Wirkpunkt an einer Position ist, an der das dritte chirurgische Instrument (113c) auf die Gewebe und Organe einwirkt.

5. Chirurgisches Robotersystem nach Anspruch 1 oder 2, wobei das Ausführungsterminal ferner einen Kraftsensor umfasst, der kommunikativ mit der Steuereinheit verbunden ist, und wobei der Kraftsensor ausgebildet ist, die kartesische Kraft zu erfassen, die von den Geweben und Organen auf das erste chirurgische Instrument (113a) ausgeübt wird.

6. Chirurgisches Robotersystem nach Anspruch 1, wobei, wenn das zweite chirurgische Instrument (113b) beginnt, eine chirurgische Operation an den Geweben und Organen durchzuführen, die Steuereinheit die Größe der kartesischen Kraft erhält, die von den Geweben und Organen auf das erste chirurgische Instrument (113a) ausgeübt wird, und den voreingestellten Kraftwert mit der Größe der kartesischen Kraft vergleicht, die von den Geweben und Organen auf das erste chirurgische Instrument (113a) ausgeübt wird, oder wobei die Steuereinheit die Größe der kartesischen Kraft, die von den Geweben und Organen auf das erste chirurgische Instrument (113a) ausgeübt wird, jederzeit erhält, wobei, wenn das zweite chirurgische Instrument (113b) beginnt, eine chirurgische Operation an den Geweben und Organen durchzuführen, die Steuereinheit den voreingestellten Kraftwert mit der Größe der kartesischen Kraft vergleicht, die von den Geweben und Organen auf das erste chirurgische Instrument (113a) ausgeübt wird.

7. Chirurgisches Robotersystem nach Anspruch 6, wobei

die Steuereinheit ferner ausgebildet ist, eine Größe einer kartesischen Kraft zu erhalten, die von dem zweiten chirurgischen Instrument (113b) empfangen wird, und die Größe der kartesischen Kraft, die von dem zweiten chirurgischen Instrument (113b) empfangen wird, mit einem Kraftschwellenwert zu vergleichen; wobei, wenn die Größe der von dem zweiten chirurgischen Instrument (113b) empfangenen kartesischen Kraft größer als der Kraftschwellenwert ist, die Steuereinheit bestimmt, dass das zweite chirurgische Instrument (113b) beginnt, eine chirurgische Operation an den Geweben und Organen durchzuführen; oder wobei die Steuereinheit ferner ausgebildet ist, eine Größe einer kartesischen Kraft zu erhalten, die von dem zweiten chirurgischen Instrument (113b) empfangen wird, und eine Differenz zwischen zwei erhaltenen kartesischen Kräften, die von dem zweiten chirurgischen Instrument (113b) empfangen werden, mit einem Kraftschwellenwert zu vergleichen; wobei, wenn die Differenz zwischen den zwei erhaltenen kartesischen Kräften, die von dem zweiten chirurgischen Instrument (113b) empfangen werden, größer als der Kraftschwellenwert ist, die Steuereinheit bestimmt, dass das zweite chirurgische Instrument (113b) beginnt, eine chirurgische Operation an den Geweben und Organen durchzuführen.

8. Chirurgisches Robotersystem nach Anspruch 2, wobei

das Ausführungsterminal ferner einen ersten Positionssensor umfasst, der kommunikativ mit der Steuereinheit verbunden ist, und wobei der erste Positionssensor ausgebildet ist, eine Position des ersten Werkzeugarms (112a) und/oder eine Position des ersten chirurgischen Instruments (113a) zu erfassen, wobei die Steuereinheit ausgebildet ist, eine Position des Wirkpunkts der kartesischen Kraft, die von den Geweben und Organen auf das erste chirurgische Instrument (113a) ausgeübt wird, gemäß der Position des ersten Werkzeugarms (112a) und/oder der Position des ersten chirurgischen Instruments (113a) zu erhalten.

9. Chirurgisches Robotersystem nach Anspruch 3, wobei

das Ausführungsterminal ferner einen ersten Positionssensor umfasst, der kommunikativ mit der Steuereinheit verbunden ist, und wobei der erste Positionssensor ausgebildet ist, eine Position des zweiten Werkzeugarms (112b) und/oder eine Position des zweiten chirurgischen Instruments (113b) zu erfassen, wobei die Steuereinheit ausgebildet ist, eine Position zu erhalten, an der das zweite chirurgische Instrument (113b) auf die Gewebe und Organe gemäß der Position des zweiten Werkzeugarms (112b) und/oder der Position des zweiten chirurgischen Instruments (113b) einwirkt.

10. Chirurgisches Robotersystem nach Anspruch 2, wobei

das Ausführungsterminal ferner einen dritten Werkzeugarm (112c) umfasst, der mit einem dritten chirurgischen Instrument (113c) versehen ist; wobei das dritte chirurgische Instrument (113c) und der dritte Werkzeugarm (112c) ausgebildet sind, Gewebe und Organe zu ziehen, wobei der voreingestellte Wirkpunkt einen ersten voreingestellten Wirkpunkt und einen zweiten voreingestellten Wirkpunkt umfasst; wobei, wenn die Größe der von den Geweben und Organen auf das erste chirurgische Instrument (113a) ausgeübten kartesischen Kraft kleiner als der voreingestellte Kraftwert ist, die Steuereinheit eine Richtung von dem ersten voreingestellten Wirkpunkt zu einem Wirkpunkt der kartesischen Kraft, die von den Geweben und Organen auf das erste chirurgische Instrument (113a) ausgeübt wird, als eine Richtung einer voreingestellten Kraft zugrunde legt, und dann die Antriebsvorrichtung weiterhin ansteuert, um den ersten Werkzeugarm (112a) und das erste chirurgische Instrument (113a) gemäß der voreingestellten Kraft anzutreiben, um die durch das erste chirurgische Instrument (113a) auf die Gewebe und Organe ausgeübte Zugkraft auf den voreingestellten Kraftwert zu erhöhen; wobei die Steuereinheit ferner ausgebildet ist, eine Größe der kartesischen Kraft, die von den Geweben und Organen auf das dritte chirurgische Instrument (113c) ausgeübt wird, mit einer voreingestellten Kraft zu vergleichen, wobei, wenn die Größe der von den Geweben und Organen auf das dritte chirurgische Instrument (113c) ausgeübten kartesischen Kraft kleiner als der voreingestellte Kraftwert ist, die Steuereinheit eine Richtung von dem zweiten voreingestellten Wirkpunkt zu einem Wirkpunkt der kartesischen Kraft, die von den Geweben und Organen auf das dritte chirurgische Instrument (113c) ausgeübt wird, als eine Richtung einer voreingestellten Kraft zugrunde legt, und die Antriebsvorrichtung ansteuert, um den dritten Werkzeugarm (112c) und das dritte chirurgische Instrument (113c) gemäß der voreingestellten Kraft anzutreiben, um die durch das dritte chirurgische Instrument (113c) auf die Gewebe und Organe ausgeübte Zugkraft auf den voreingestellten Kraftwert zu erhöhen; wobei der erste voreingestellte Wirkpunkt an einer Position ist, an der das dritte chirurgische Instrument (113c) auf die Gewebe und Organe einwirkt, und wobei der zweite voreingestellte Wirkpunkt an einer Position ist, an der das erste chirurgische Instrument (113a) auf die Gewebe und Organe einwirkt.

11. Chirurgisches Robotersystem nach Anspruch 1, ferner umfassend ein Steuerterminal, wobei das Steuerterminal einen Hauptmanipulator umfasst, der ausgebildet ist, eine Bewegung des ersten Werkzeugarms (112a) und des ersten chirurgischen Instruments (113a) zu steuern und die kartesische Kraft, die von den Geweben und Organen auf das erste chirurgische Instrument (113a) ausgeübt wird, zu empfangen; und wobei der voreingestellte Kraftwert basierend auf der kartesischen Kraft bestimmt wird, die von den Geweben und Organen auf das erste chirurgische Instrument (113a) ausgeübt wird, wenn die Gewebe und Organe gespannt sind.

## Revendications

1. Système de robot chirurgical, comprenant un terminal d'exécution, le terminal d'exécution comprenant un premier bras d'outil (112a) pourvu d'un premier instrument chirurgical (113a), un deuxième bras d'outil (112b) pourvu d'un deuxième instrument chirurgical (113b), un dispositif d'entraînement configuré pour entraîner le premier bras d'outil (112a), le premier instrument chirurgical (113a), le deuxième bras d'outil (112b) et le deuxième instrument chirurgical (113b) à se déplacer, et une unité de commande connectée de manière communicative avec le dispositif d'entraînement,

le premier bras d'outil (112a) et le premier instrument chirurgical (113a) étant configurés pour retirer des tissus et organes ;

le deuxième bras d'outil (112b) et le deuxième instrument chirurgical (113b) étant configurés pour effectuer une opération chirurgicale sur des tissus et organes ;

l'unité de commande étant configurée pour obtenir une amplitude d'une force cartésienne exercée par les tissus et organes sur le premier instrument chirurgical (113a), et comparer l'amplitude de la force cartésienne exercée par les tissus et organes sur le premier instrument chirurgical (113a) avec une valeur de force prédéfinie ; **caractérisé en ce que** si l'amplitude de la force cartésienne exercée par les tissus et organes sur le premier instrument chirurgical (113a) est inférieure à la valeur de force prédéfinie, l'unité de commande commande le dispositif d'entraînement d'entraîner le premier bras d'outil (112a) et le premier instrument chirurgical (113a) en fonction de la valeur de force prédéfinie, pour augmenter une force de traction exercée par le premier instrument chirurgical (113a) sur les tissus et organes jusqu'à la valeur de force prédéfinie et obtenir une retrait autonome.

2. Système de robot chirurgical selon la revendication 1, si l'amplitude de la force cartésienne exercée par les tissus et organes sur le premier instrument chirurgical (113a) est inférieure à la valeur de force prédéfinie, l'unité de commande prenant une direction d'un point d'action prédéfini à un point d'action de la force cartésienne exercée par les tissus et organes sur le premier instrument chirurgical (113a) comme une direction d'une force prédéfinie, et commandant le dispositif d'entraînement d'entraîner le premier bras d'outil (112a) et le premier instrument chirurgical (113a) en fonction de la force prédéfinie, pour augmenter la force de traction exercée par le premier instrument chirurgical (113a) sur les tissus et organes jusqu'à la valeur de force prédéfinie.

3. Système de robot chirurgical selon la revendication 2, le point d'action prédéfini se trouvant à une position où le deuxième instrument chirurgical (113b) agit initialement sur les tissus et organes, ou bien le point d'action prédéfini étant à une position où le deuxième instrument chirurgical (113b) agit sur les tissus et organes lorsque l'unité de commande obtient le point d'action prédéfini.

4. Système de robot chirurgical selon la revendication 2, le terminal d'exécution comprenant en outre un troisième bras d'outil (112c) pourvu d'un troisième instrument chirurgical (113c) ; le troisième bras d'outil (112c) et le troisième instrument chirurgical (113c) étant configurés pour serrer des tissus et organes, et le point d'action prédéfini étant à une position où le troisième instrument chirurgical (113c) agit sur les tissus et organes.

5. Système de robot chirurgical selon la revendication 1 ou 2, le terminal d'exécution comprenant en outre un capteur de force connecté de manière communicative avec l'unité de commande, et le capteur de force étant configuré pour détecter la force cartésienne exercée par les tissus et organes sur le premier instrument chirurgical (113a).

6. Système de robot chirurgical selon la revendication 1, lorsque le deuxième instrument chirurgical (113b) commence à effectuer une opération chirurgicale sur les tissus et organes, l'unité de commande obtenant l'amplitude de la force cartésienne exercée par les tissus et organes sur le premier instrument chirurgical (113a), et comparant la valeur de force prédéfinie avec l'amplitude de la force cartésienne exercée par les tissus et organes sur le premier instrument chirurgical (113a), ou l'unité de commande obtenant l'amplitude de la force cartésienne exercée toujours par les tissus et organes sur le premier instrument chirurgical (113a), lorsque le deuxième instrument chirurgical (113b) commence à effectuer une opération chirurgicale sur les tissus et organes, l'unité de commande comparant la valeur de force prédéfinie avec l'amplitude de la force cartésienne exercée par les tissus et organes sur le premier instrument chirurgical (113a).

7. Système de robot chirurgical selon la revendication 6,

l'unité de commande étant également configurée pour obtenir une amplitude d'une force cartésienne reçue par le deuxième instrument chirurgical (113b), et comparer l'amplitude de la force cartésienne reçue par le deuxième instrument chirurgical (113b) avec un seuil de force ; si l'amplitude de la force cartésienne reçue par le deuxième instrument chirurgical (113b) est supérieure au seuil de force, l'unité de commande déterminant que le deuxième instrument chirurgical (113b) commence à effectuer une opération chirurgicale sur les tissus et organes ; ou l'unité de commande étant également configurée pour obtenir une amplitude d'une force cartésienne reçue par le deuxième instrument chirurgical (113b), et comparer une différence entre deux forces cartésiennes obtenues reçues par le deuxième instrument chirurgical (113b) avec un seuil de force ; si la différence entre les deux forces cartésiennes obtenues reçues par le deuxième instrument chirurgical (113b) est supérieure au seuil de force, l'unité de commande déterminant que le deuxième instrument chirurgical (113b) commence à effectuer une opération chirurgicale sur les tissus et organes.

8. Système de robot chirurgical selon la revendication 2,

le terminal d'exécution comprenant en outre un premier capteur de position connecté de manière communicative avec l'unité de commande, et le premier capteur de position étant configuré pour détecter une position du premier bras d'outil (112a) et/ou une position du premier instrument chirurgical (113a),
l'unité de commande étant configurée pour obtenir une position du point d'action de la force cartésienne exercée par les tissus et organes sur le premier instrument chirurgical (113a) en fonction de la position du premier bras d'outil (112a) et/ou de la position du premier instrument chirurgical (113a).

9. Système de robot chirurgical selon la revendication 3,

le terminal d'exécution comprenant en outre un premier capteur de position connecté de manière communicative avec l'unité de commande, et le premier capteur de position étant configuré pour détecter une position du deuxième bras d'outil (112b) et/ou une position du deuxième instrument chirurgical (113b),
l'unité de commande étant configurée pour obtenir une position où le deuxième instrument chirurgical (113b) agit sur les tissus et organes en fonction de la position du deuxième bras d'outil (112b) et/ou de la position du deuxième instrument chirurgical (113b).

10. Système de robot chirurgical selon la revendication 2,

le terminal d'exécution comprenant en outre un troisième bras d'outil (112c) pourvu d'un troisième instrument chirurgical (113c) ; le troisième instrument chirurgical (113c) et le troisième bras d'outil (112c) étant configurés pour retirer des tissus et organes,
le point d'action prédéfini comprenant un premier point d'action prédéfini et un deuxième point d'action prédéfini ;
si l'amplitude de la force cartésienne exercée par les tissus et organes sur le premier instrument chirurgical (113a) est inférieure à la valeur de force prédéfinie, l'unité de commande prenant une direction allant du premier point d'action prédéfini à un point d'action de la force cartésienne exercée par les tissus et organes sur le premier instrument chirurgical (113a) comme direction d'une force prédéfinie,
puis continuant à commander le dispositif d'entraînement d'entraîner le premier bras d'outil (112a) et le premier instrument chirurgical (113a) en fonction de la force prédéfinie, pour augmenter la force de traction exercée par le premier instrument chirurgical (113a) sur les tissus et organes jusqu'à la valeur de force prédéfinie ;
l'unité de commande étant en outre configurée pour comparer une amplitude de la force cartésienne exercée par les tissus et organes sur le troisième instrument chirurgical (113c) à la valeur de force prédéfinie ; si l'amplitude de la force cartésienne exercée par les tissus et organes sur le troisième instrument chirurgical (113c) est inférieure à la valeur de force prédéfinie, l'unité de commande prenant une direction allant du deuxième point d'action prédéfini à un point d'action de la force cartésienne exercée par les tissus et organes sur le troisième instrument chirurgical (113c) comme direction d'une force prédéfinie, et commandant le dispositif d'entraînement d'entraîner le troisième bras d'outil (112c) et le troisième instrument chirurgical (113c) en fonction de la force prédéfinie, pour augmenter une force de traction exercée par le troisième instrument chirurgical (113c) sur les tissus et organes jusqu'à la valeur de force prédéfinie ;
le premier point d'action prédéfini étant à une position où le troisième instrument chirurgical (113c) agit sur les tissus et organes, et le deuxième point d'action prédéfini étant à une position où le premier instrument chirurgical (113a) agit sur les tissus et organes.

11. Système de robot chirurgical selon la revendication 1, comprenant en outre un terminal de commande, le terminal de commande comprenant un manipulateur principal configuré pour commander un mouvement du premier bras d'outil (112a) et du premier instrument chirurgical (113a), et recevoir la force cartésienne exercée par les tissus et organes sur le premier instrument chirurgical (113a) ; et la valeur de force prédéfinie étant déterminée sur la base de la force cartésienne exercée par les tissus et organes sur le premier instrument chirurgical (113a) lorsque les tissus et organes sont tendus.

Fig. 1

Fig. 2

Fig. 3

Fig. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 106028994 A
- CN 107427327 A
- CN 108472087 A
- EP 3260051 A1
- US 2012182134 A1
- US 9833254 B1
- KR 101742534 B1
- CN 105232155 A